# FASCICULE DE BREVET EUROPEEN

(11) **EP 1 646 379 B1**
(45) Date de publication et mention de la délivrance du brevet: **18.09.2013**
(21) Numéro de dépôt: 04767624.2
(22) Date de dépôt: 08.07.2004
(51) Int. Cl.: A61K 31/40, A61K 9/00, A61K 9/50, A61K 9/20

(54) **NOUVELLE COMPOSITION PHARMACEUTIQUE SOLIDE COMPRENANT DE L AMISULPRIDE**
NEUE FESTE PHARMAZEUTISCHE ZUSAMMENSETZUNG MIT AMISULPRID
NOVEL SOLID PHARMACEUTICAL COMPOSITION COMPRISING AMISULPRIDE

(30) Priorité: 09.07.2003 FR 0308409
(43) Date de publication de la demande: 19.04.2006
(73) Titulaire: SANOFI, 75008 Paris (FR)
(72) Inventeur: ANDRE, Frédéric, FR-91370 Verrières-Le-Buisson (FR); DA CRUZ, Henri, F-77350 Le Mee-Sur-Seine (FR); DENNI, Michel, F-91420 Morangis (FR); LEWIS, Gareth, F-91410 Dourdan (FR); MIGNONNEAU, Jérôme, F-49350 Saint-Clément-des-Levées (FR); RIBARDIERE, Agnès, F-92260 Fontenay-Aux-Roses (FR)
(74) Mandataire: Weber, Mathieu
(86) Numéro de dépôt international: PCT/FR2004/001792
(87) Numéro de publication internationale: WO 2005/004860

(56) Documents cités:
- WO-A-00/51563

## Description

La présente invention se rapporte à une nouvelle composition pharmaceutique solide comprenant de l'amisulpride, cette composition étant sous forme orodispersible.

L'amisulpride ou 4-amino-N-[(1-éthyl-2-pyrrolidin-yl)méthyl]-5-(éthylsulfonyl)-2-méthoxybenzamide, ses isomères et certains de leurs dérivés sont décrits en particulier dans le fascicule publié du brevet américain US-4,401,822 de Thominet et al.

L'amisulpride est un antipsychotique atypique utilisé dans le traitement des psychoses, plus particulièrement dans le traitement des schizophrénies paranoïdes et productives, des psychoses délirantes aiguës, ainsi que dans le traitement des états déficitaires des schizophrénies, des évolutions psychotiques résiduelles et des états d'inhibition avec ralentissement. L'amisulpride est également utilisé dans le traitement de symptômes négatifs primaires prédominants. L'amisulpride est également utile dans le traitement de la dysthymie.

L'amisulpride est connu pour être administré par voie orale, généralement sous forme de comprimés dosés à 100, 200 ou 400 mg chacun (Vidal, édition 2003, monographie Solian® aux pages 1736 et 1738).

Les doses quotidiennes en amisulpride administrées par voie orale sont toutefois souvent élevées et peuvent aller jusqu'à 1200 mg/jour lors des épisodes psychotiques aigus.

Les patients traités par l'amisulpride doivent ainsi absorber plusieurs comprimés quotidiennement.

Or, certains de ces patients, en raison même de leur état pathologique, peuvent rencontrer des difficultés, voire montrer une réticence marquée à absorber régulièrement un nombre élevé de comprimés et donc à observer correctement leur prescription.

On a alors cherché à mettre au point de nouvelles formes orales d'amisulpride.

Il a tout d'abord été envisagé de développer des comprimés comprenant une dose plus forte d'amisulpride, par exemple des doses supérieures à 600 mg. Toutefois, de tels comprimés se sont avérés présenter une taille trop importante pour être facilement avalés par les patients. Des études ont montré qu'une gamme de comprimés dosés à 100 mg, 200 mg et 400 mg d'amisulpride restait la plus appropriée.

Il a ensuite été envisagé de prolonger et/ou d'intensifier l'absorption gastro-intestinale de l'amisulpride en vue, notamment, de diminuer le nombre de prises quotidiennes de comprimés d'amisulpride, tout en maintenant, voire en améliorant, l'efficacité thérapeutique de ce principe actif. On peut en particulier citer WO 00/51563 qui décrit une composition comprenant une association d'un benzamide (notamment l'amisulpride) et d'un promoteur d'absorption (notamment un inhibiteur de la P-glycoproteine), cette association étant destinée à améliorer l'absorption intestinale du benzamide, c'est-à-dire à augmenter la f raction d e benzamide f ranchissant la b arrière intestinale (page 1, lignes 21-23), et ainsi à pouvoir réduire les doses administrées pour une dose efficace donnée et réduire le nombre de prises quotidiennes (page 2, lignes 23-26).

Toutefois, quel que soit le nombre de prise, on a constaté que le problème principal pour le principe actif amisulpride consiste à pouvoir assurer la bonne prise des comprimés par les patients.

Ce problème d'observance est en effet tout à fait crucial, compte tenu des pathologies spécifiques à traiter, en particulier chez les patients les plus réticents nécessitant l'intervention d'une aide extérieure pour assurer la bonne qualité de la prise, c'est à dire la déglutition effective du comprimé d'amisulpride.

En effet, on a constaté que les patients réticents ont tendance à vouloir simuler la prise du comprimé d'amisulpride, notamment en avalant l'eau qu'on leur présente tout en maintenant le comprimé caché dans leur cavité buccale, pour s'en débarrasser ultérieurement en le recrachant.

Or, tout particulièrement en milieu hospitalier, on ne peut consacrer qu'un temps limité par patient pour cette aide extérieure, ce qui rend plus facile ce type de simulation constituant donc un réel problème pour la santé du patient.

Un autre problème lié à ce principe actif amisulpride réside en son amertume très importante.

Afin de pouvoir quantifier le degré d'importance de cette amertume, un panel de 6 personnes a permis de constater que l'amertume de l'amisulpride est détectée avec un comprimé contenant 1 mg d'amisulpride libérable dans la bouche. Cette amertume devient inacceptable quand le comprimé contient 8 mg d'amisulpride libérable dans la bouche.

On mesure ainsi le degré d'intérêt d'une administration d'amisulpride à des doses de 100 à 400 mg par voie orale, qui permettrait d'éviter toute simulation sans présenter toutefois une sensation d'amertume en bouche rédhibitoire à toute déglutition, afin d'obtenir une amélioration réelle de l'observance en particulier chez les patients les plus réticents.

Ce problème d'observance doit toutefois également être pris en compte chez les patients les moins réticents que les thérapeutes sont parvenus à stabiliser après souvent plusieurs mois d'un traitement correctement suivi.

En effet, il est admis par les thérapeutes des patients déjà traités par l'amisulpride, en particulier des patients schizophrènes, qu'un simple changement d'apparence ou des modalités de prise du médicament peut entraîner une déstabilisation dangereuse de ces patients.

Enfin, il est également admis par les thérapeutes que tout changement dans la composition pharmaceutique pour un principe actif comme amisulpride devrait de préférence permettre de conserver, outre l'apparence du médicament, la pharmacocinétique avantageusement procurée par les comprimés déjà utilisés par les patients afin de prévenir toute déstabilisation. Or, on a pu constater que le principe actif amisulpride, lorsqu'il doit être administré par voie orale, présente une biodisponibilité limitée (de l'ordre de 48 %) qui peut être attribuée à une absorption partielle de ce principe actif au niveau gastro-intestinal. Ainsi, certaines modifications du profil de libération pourraient entraîner un passage insuffisant au niveau cérébral.

On a maintenant trouvé de manière tout à fait inattendue et surprenante une nouvelle composition pharmaceutique solide destinée à l'administration par voie orale d'amisulpride, permettant de résoudre les problèmes spécifiques de ce principe actif.

Cette nouvelle composition constitue en particulier une réelle amélioration de la prise en charge de tous les patients concernés, dans la mesure où elle permet d'améliorer de manière significative l'observance par les patients les plus réticents tout en restant utilisable par les patients les moins réticents.

La présente invention se rapporte ainsi à une nouvelle composition pharmaceutique solide pour administration par voie orale du principe actif amisulpride, caractérisée en ce qu'elle comprend au moins une particule d'amisulpride enrobée et au moins un excipient pharmaceutiquement acceptable adapté à une administration orodispersible de la composition.

Par « administration orodispersible », on entend selon la présente invention une administration orale par délitement rapide de la composition solide (notamment un comprimé) dans la bouche, de préférence en moins de 30 secondes, sans nécessité de mastication ni d'apport d'eau pour avaler la composition délitée, la composition devant pouvoir être avalée de préférence en moins de 90 secondes après la mise en bouche. Par « administration orodispersible », on entend ainsi en particulier selon la présente invention une administration orale par délitement buccal de la composition solide avant d'être avalée par le patient, par opposition à une administration orale « classique » c'est-à-dire une administration orale où le patient avale une composition solide n on délitée (notamment un comprimé classique) avant le début d'un délitement éventuel par exemple dans l'estomac. L'homme du métier comprend ainsi en particulier que l'enveloppe d'une gélule classique ou encore un sachet ne peuvent être considérés comme un excipient pharmaceutiquement acceptable adapté à une telle administration orodispersible.

Par « amisulpride », on entend selon la présente invention le (R,S)-amisulpride, c'est à dire le mélange racémique (par exemple celui fabriqué par la société Finorga), présent dans la forme pharmaceutique distribuée par la société SANOFI-SYNTHELABO sous la dénomination SOLIAN®, un isomère levogyre ou dextrogyre de l'amisulpride, un dérivé de l'amisulpride ou de l'un de ses isomères levogyre et dextrogyre, ce dérivé étant choisi parmi un sel d'addition avec un acide pharmaceutiquement acceptable, un sel d'ammonium quaternaire ou un oxyde, de l'amisulpride ou de l'un de ses isomères levogyre et dextrogyre, ou un mélange de ceux-ci.

Par « particule d'amisulpride », on entend selon la présente invention une particule comprenant de l'amisulpride, cette particule pouvant être sous forme sphérique ou sphéroïdale, parfaite ou non, ou sous toute autre forme de particule connue de l'homme du métier, telle qu'obtenue par un procédé de granulation (granulation humide, granulation avec un liant fusible, granulation par montage avec des poudres et/ou des liquides, par rotogranulation et granulation par extrusion-sphéronisation), un procédé de fluidisation, un procédé d'atomisation, un procédé de prilling ou par un procédé de cristallisation d'amisulpride. L'homme du métier comprend ainsi que cette particule d'amisulpride peut ne comprendre que de l'amisulpride, notamment lorsqu'elle est obtenue par un procédé de cristallisation, ou peut comprendre en outre au moins un agent résultant de la mise en oeuvre du procédé de formation de cette particule d'amisulpride, notamment un excipient de sphéronisation lorsque cette particule d'amisulpride est obtenue par un procédé de granulation, comme décrit ci-après.

La composition solide selon l'invention peut bien entendu comprendre plus d'une particule d'amisulpride enrobée, sous forme d'un mélange avec ledit au moins un excipient pharmaceutiquement acceptable adapté à une administration orodispersible de la composition.

La particule d'amisulpride enrobée est de préférence constituée d'une particule d'amisulpride, telle que définie ci-dessus, présentant à sa surface un enrobage choisi dans le groupe constitué par les enrobages lipidiques et les enrobages polymères.

Les procédés d'enrobages lipidiques et polymères pouvant être utilisés pour l'enrobage de particules d'amisulpride sont bien connus de l'homme du métier.

L'enrobage lipidique, également connu sous la dénomination « enrobage hot-melt » est un procédé d'enrobage à chaud d'une particule par pulvérisation sur cette dernière d'une matière d'enrobage thermofusible, par exemple une huile ou une cire. Il peut être mis en oeuvre de façon avantageuse dans un appareil à lit d'air fluidisé notamment de type GPCG1 tel que commercialisé par la société Glatt..

L'enrobage polymére, réalisé de manière connue avec des véhicules aqueux ou organiques, est un procédé d'enrobage d'une particule par pulvérisation sur cette dernière d'une solution ou d'une dispersion aqueuse ou organique d'un polymère et d'additifs connus tels qu'un plastifiant, notamment l'acide stéarique, le triethyl citrate et/ou le dibutyl sebaçate, et/ou un anti-adhérent tel que le talc, un dérivé de silice, tel que le Syloid® (gel de silice), ou le stéarate de magnésium. L'étape d'enrobage peut être suivie d'une étape de maturation permettant l'évaporation des solvants résiduels et l'achèvement de la formation du film d'enrobage. Ce procédé d'enrobage polymère peut être mis en oeuvre dans un appareil à lit d'air fluidisé.

Lorsque la particule d'amisulpride comprend un enrobage lipidique, cet enrobage lipidique comprend de préférence une matière lipidique choisie dans le groupe constitué par:
- les mono-, di- et triglycérides d'acides gras formés de chaînes ayant de 6 à 32 atomes de carbone, de préférence saturées, tels que le monostéarate de glycérol, le distéarate de glycérol, l'huile de coton hydrogénée, l'huile de palme hydrogénée, l'huile de soja hydrogénée, l'huile de ricin hydrogénée et plus généralement les huiles végétales hydrogénées. L'huile de ricin hydrogénée, connue sous la dénomination glyceryl-tri-(12-hydroxystearate) (RN CAS 8001-78-3), est particulièrement préférée, telle que celle commercialisée sous la dénomination CUTINA HR® ;
- les monoesters et diesters de polyglycérols, tels que le monostéarate de diglycérol, le monostéarate de triglycérol, le dipalmitate d'hexaglycérol, et le distéarate de décaglycérol ;
- les monoesters et diesters de polyéthylène glycol, en particulier les mélanges de ces derniers avec des mono-, di- et triglycérides, tels que ceux commercialisés sous les dénominations respectives « Gelucire 50/13^{®} » et « Gelucire 50/02^{®} » par la société Gattefossé. On peut plus particulièrement utiliser un mélange de « Gelucire 50/13® » et de « Gelucire 50/02® ;
- les esters de propylène glycol, tels que le monostéarate de propylène glycol ;
- les esters d'acides gras de sucrose, formés de chaînes ayant de 6 à 32 atomes de carbone, tels que le monostéarate de sucrose, le monopalmitate de sucrose ;
- les acides gras et les alcools gras, formés de chaînes ayant de 6 à 32 atomes de carbone, tels que l'acide stéarique, l'alcool cétylique ou l'alcool stéarylique ;
- les cires végétales, les cires animales, les cires de pétrole, les cires synthétiques, telles que la cire de jojoba, la cire de carnauba, le beurre de cacao, la cire d'abeille, la lanoline, la cire de paraffine,
et les mélanges de ceux-ci.

Lorsque la particule d'amisulpride comprend un enrobage polymère, ce dernier comprend de préférence un polymère choisi dans le groupe constitué par les polymères cellulosiques, les polymères acryliques, les polymères vinyliques, les polymères carboxyvinyliques, et les mélanges de ceux-ci.

Lorsque l'enrobage polymère comprend un polymère cellulosique, ce dernier peut être avantageusement choisi dans le groupe constitué par l'éthylcellulose, l'hydroxyéthylcellulose, l'hydroxypropylcellulose, l'hydroxypropylméthylcellulose, la carboxyméthylcellulose, et les mélanges de ceux-ci. On peut utiliser en particulier de l'éthylcellulose en dispersion aqueuse, telle que celle commercialisée sous la dénomination Aquacoat® par la société FMC BioPolymer.

De préférence, l'enrobage polymère comprend un polymère acrylique choisi dans le groupe constitué par les polymères acryliques, les polymères méthacryliques, les copolymères ammonio-méthacrylates (notamment les Eudragit® RL et RS), les polyacrylates (notamment l'Eudragit® NE), les polyméthacrylates (notamment l' Eudragit® E), et les mélanges de ceux-ci.

La particule d'amisulpride enrobée présente dans la composition selon l'invention peut également avantageusement être obtenue par un procédé comprenant au moins une étape de microencapsulation de particule d'amisulpride. La microencapsulation peut être obtenue par des procédés bien connus de l'homme du métier, notamment par coacervation (séparation de phase).

Bien entendu, la composition selon l'invention peut comprendre un mélange de différentes particules d'amisulpride enrobées choisies parmi celles pouvant être obtenues par microencapsulation, par enrobage lipidique et/ou par enrobage polymère, comme décrit ci-dessus.

La particule d'amisulpride enrobée présente dans la composition solide selon l'invention présente de préférence une taille comprise e ntre e nviron 21 µm et environ 1000 µm, en particulier une taille comprise entre environ 31 µm et environ 800 µm et plus particulièrement une taille comprise entre environ 91 µm et environ 550 µm.

La proportion d'enrobage dans une particule d'amisulpride enrobée est de préférence comprise entre environ 1 et environ 50 % en poids , plus particulièrement entre environ 7 et environ 35 % en poids, par rapport au poids total de particule d'amisulpride enrobée.

La proportion d'amisulpride dans une particule d'amisulpride enrobée est de préférence comprise entre environ 40 et environ 99 % en poids, par rapport au poids total de particule d'amisulpride enrobée.

Ces proportions respectives d'enrobage et d'amisulpride peuvent être en particulier c hacune d éterminée par l'homme d u m étier c ompte tenu d es c onditions d u procédé de préparation de la particule d'amisulpride enrobée, comme illustré dans les exemples ci-après.

La particule d'amisulpride enrobée présent dans la composition solide selon l'invention est constituée d'une particule d'amisulpride présentant de préférence une taille comprise entre environ 20 µm et environ 700 µm, en particulier une taille comprise entre environ 30 µm et environ 600 µm et plus particulièrement une taille comprise entre environ 90 µm et environ 500 µm, tel que mesuré par tamisage à l'issue du procédé de préparation de la particule d'amisulpride.

La particule d'amisulpride utilisée pour la composition selon l'invention, telle que déjà définie ci-dessus, peut être préparée selon des méthodes connues de l'homme du métier, en particulier par granulation ou par cristallisation.

La granulation peut ainsi être en particulier une granulation humide, une granulation avec un liant fusible, une granulation par montage avec des poudres et/ou des liquides, une rotogranulation ou une granulation par extrusion-sphéronisation. Ces principales voies de granulation reposent sur les principes suivants : agglomération ou montage par pulvérisation de poudre ou de liquide.

Pour la p réparation d es p articules d'amisulpride d e la c omposition solide selon l'invention, on préfère utiliser un procédé de granulation humide ou un procédé de granulation par extrusion-sphéronisation.

Selon un mode de réalisation préféré de la présente invention, la particule d'amisulpride est un sphéroïde comprenant de l'amisulpride, obtenu par granulation.

Le sphéroïde d'amisulpride comprend ainsi de préférence en outre au moins un excipient de sphéronisation choisi dans le groupe constitué par les agents liants, les agents diluants, les tensioactifs, et les mélanges de ceux-ci.

Parmi les agents liants préférés pour la granulation de l'amisulpride, on peut citer la povidone (ou polyvinylpyrrolidone; PVP), en particulier pour une granulation humide, telle que la povidone K30, les polymères cellulosiques, en particulier pour une granulation par extrusion-sphéronisation, tels que l'hydroxypropylcellulose commercialisée sous la dénomination Klucel JF® par la société Aqualon, les gommes, les polymères acryliques, les polyéthylène glycols (PEG), la gélatine et les matières lipidiques. Parmi les agents diluants préférés, en particulier pour une granulation par extrusion-sphéronisation, on peut citer la cellulose microcristalline telle que celle commercialisée sous la dénomination Avicel PH101 ® par la société FMC BioPolymer. Parmi les tensioactifs connus de l'homme du métier pour leur utilisation en granulation, en particulier par extrusion-sphéronisation, on peut citer le laurylsulfate de sodium.

Selon un autre mode de réalisation de la présente invention, la particule d'amisulpride est susceptible d'être obtenue par un procédé comprenant au moins une étape de cristallisation d'amisulpride, telle que la cristallisation sphérique.

Compte tenu de ce qui précède, l'homme du métier comprend que l'enrobage de la particule d'amisulpride enrobée tel que décrit ci-dessus permet avantageusement de masquer le goût de l'amisulpride, en particulier son amertume. En d'autres termes, selon l'invention, ladite au moins une particule d'amisulpride enrobée est constituée d'une particule d'amisulpride présentant à sa surface un enrobage pour masquer le goût de l'amisulpride.

De préférence, un tel enrobage n'a pas d'effet significatif sur la libération immédiate de l'amisulpride, c'est à dire que la cinétique de libération *in vivo* du principe actif (amisulpride) n'est pas substantiellement modifiée avec la composition selon l'invention par rapport à celle des compositions pharmaceutiques à libération immédiate d'amisulpride déjà utilisées par les patients. En d'autres termes, selon l'invention, de préférence, ladite au moins une particule d'amisulpride enrobée est constituée d'une particule d'amisulpride présentant à sa surface un enrobage pour masquer le goût de l'amisulpride sans substantiellement modifier la libération de l'amisulpride.

Ainsi, de préférence, la composition selon l'invention est une composition à libération immédiate d'amisulpride.

Par « composition à libération immédiate d'amisulpride », on entend selon l'invention une composition permettant une dissolution in vitro d'au moins 80 % en poids d'amisulpride en moins de 30 minutes dans un tampon aqueux à 0,1 M d'acide chlorhydrique à 37°C, avec un appareil de dissolution à palette (agitation à 100 tours/minute), conformément au test de dissolution in vitro décrit dans la Pharmacopée européenne section 4.4.

L'excipient pharmaceutiquement acceptable adapté à une administration orodispersible de la composition solide selon l'invention (ci-après également « excipient pour administration orodispersible ») est de préférence choisi dans le groupe constitué par les agents désintégrants, les agents de salivation, les agents diluants à propriétés liantes, et les mélanges de ceux-ci.

Parmi les agents désintégrants, on peut citer en particulier la crospovidone (ou polyvinylpyrrolidone réticulée), la croscarmellose (ou carboxyméthylcellulose sodique réticulée), les amidons et leurs dérivés tels que les amidons modifiés prégélatinisés et le carboxyméthylamidon, les dérivés d'hydroxypropylcellulose faiblement substitués, l'acide alginique et ses sels, et les mélanges de ceux-ci.

Parmi les agents de salivation, on peut citer en particulier l'acide citrique et les sels d'acide citrique, tel que l'acide citrique anhydre granulé.

Parmi les agents diluants à propriétés liantes, on peut citer en particulier les polyols, plus particulièrement le mannitol, le sorbitol, le lactitol, le xylitol, l'érythritol et dérivés, le maltitol, et les mélanges de ceux-ci, notamment le xylitollsorbitol coatomisé.

L'excipient pharmaceutiquement acceptable adapté à une administration orodispersible de la composition est de préférence présent selon une proportion comprise entre environ 2 et environ 90 % en poids, plus particulièrement une proportion comprise entre environ 3 et environ 85 % en poids, par rapport au poids total de la composition.

Plus particulièrement, les proportions respectives d'agent désintégrant, d'agent de salivation et d'agent diluant à propriétés liantes pouvant constituer l'excipient pharmaceutiquement acceptable adapté à une administration orodispersible de la composition selon l'invention sont, de préférence :
- d'environ 2 à environ 50 % en poids (de préférence d'environ 2 à environ 20 % en poids) d'agent désintégrant ;
- d'environ 1 à environ 15 % en poids (de préférence d'environ 1 à environ 5 % en poids) d'agent de salivation ; et/ou
- d'environ 1 à environ 90 % en poids d'agent diluant à propriétés liantes,
par rapport au poids total de la composition solide selon l'invention.

De manière avantageuse, la composition solide selon l'invention peut en outre comprendre, avec ladite au moins une particule d'amisulpride enrobée et ledit au moins un excipient pharmaceutiquement acceptable adapté à une administration orodispersible de la composition, au moins un excipient pharmaceutiquement acceptable adapté au masquage de goût (ci-après également « excipient pour masquage de goût »), tels que ceux connus de l'homme du métier.

De préférence, cet excipient pharmaceutiquement acceptable adapté au masquage de goût est choisi dans le groupe constitué par les arômes naturels, les arômes de synthèse, les édulcorants de synthèse, et les mélanges de ceux-ci. De manière avantageuse, on peut utiliser un édulcorant de synthèse sous forme de poudre ou granulé, notamment de l'aspartame.

La proportion d'excipient pharmaceutiquement acceptable adapté au masquage de goût est de préférence comprise entre environ 0,1 et environ 6 % en poids, plus particulièrement entre environ 1 et environ 5 % en poids, par rapport au poids total de la composition.

Enfin, la composition solide selon l'invention peut en outre comprendre de manière avantageuse au moins un excipient pharmaceutiquement acceptable tels que ceux connus de l'homme du métier pour la préparation par compression, la conservation et/ou l'administration de formes solides pharmaceutiques (ci-après « excipient pour forme solide »), telles que des comprimés ou des pastilles.

Cet excipient pour forme solide est en particulier choisi dans le groupe constitué par les agents diluants, les agents lubrifiants, les agents d'écoulement, les agents mouillants, les agents colorants, les agents modificateurs de pH, les agents liants, et les mélanges de ceux-ci.

Plus particulièrement, parmi les agents diluants préférés, on peut citer la cellulose microcristalline, telle que celle commercialisée sous la dénomination Avicel PH105®, le lactose t el que celui c ommercialisé sous la dénomination Ludipress® et/ou la glycine telle que la Glycine BN 500®. Le Ludipress® est un excipient pour compression directe, mélange de lactose (93%), de PVP (3,5%) et de PVP réticulée (3,5%). Parmi les agents lubrifiants préférés, on peut citer le stéarate de magnésium. Enfin, parmi les agents d'écoulement préférés, on peut citer la silice colloïdale telle que celle commercialisée sous la dénomination Aerosil® par la société Degussa . Parmi les agents liants préférés, on peut citer le polyéthylène glycol, la povidone (ou polyvinylpyrrolidone ; PVP), les polymères cellulosiques tels que l'hydroxypropylméthylcellulose (HPMC) ou l'hydroxypropylcellulose (HPC), l'éthylcellulose, les polymères acryliques tels que ceux commercialisés sous la dénomination d'Eudragit®, les huiles végétales, les gommes, la gélatine.

Comme déjà défini ci-dessus, l'amisulpride compris dans la composition pharmaceutique selon l'invention est choisi dans le groupe constitué par le (R,S)-amisulpride, les isomères levogyre et dextrogyre de l'amisulpride, les dérivés de l'amisulpride ou de l'un de ses isomères levogyre et dextrogyre, ces dérivés étant choisis parmi les sels d'addition avec un acide pharmaceutiquement acceptable, les sels d'ammonium quaternaire et les oxydes, de l'amisulpride ou de l'un de ses isomères levogyre et dextrogyre, et les mélanges de ceux-ci.

De préférence, l'amisulpride compris dans la composition pharmaceutique selon l'invention est le (R,S)-amisulpride.

L'amisulpride est présent dans la composition selon une proportion comprise entre environ 5 et environ 80 % en poids, par rapport au poids total de la composition.

De préférence, la composition solide selon l'invention comprend, par rapport au poids total de la composition :
- environ 1 0 à e nviron 4 0 % e n poids d e (*R*,*S*)-amisulpride sous forme d'au moins une particule d'amisulpride enrobée;
- environ 3 à environ 85 % en poids d'excipient pour administration orodispersible;
- environ 1 à environ 5 % en poids d'excipient pour masquage de goût ;
- environ 1 à environ 86 % en poids d'excipient pour forme solide.

La composition solide selon l'invention est de préférence sous la forme d'un comprimé.

Ainsi, selon un mode de réalisation préféré de la présente invention, la composition pharmaceutique solide selon l'invention pour administration par voie orale du principe actif amisulpride est caractérisée en ce qu'elle comprend au moins une particule d'amisulpride enrobée, au moins un excipient pharmaceutiquement acceptable adapté à une administration orodispersible de la composition, au moins excipient pour forme solide, comme défini ci-dessus, et en ce qu'elle se présente sous la forme d'un comprimé.

La composition solide selon l'invention est de préférence sous la forme d'un comprimé orodispersible comprenant de l'amisulpride selon un dosage compris entre 50 et 800 mg, en particulier entre 100 et 400 mg. Plus particulièrement, ce comprimé orodispersible peut comprendre de l'amisulpride selon un dosage de 100 mg, 200 mg ou 400 mg.

On peut préparer de tels comprimés d'amisulpride orodispersibles par compression directe, par exemple sur une machine rotative type SVIAC PR12, avec de préférence une force de compression inférieure à 600 daN pour un poinçon 16mm.

La présente composition présente ainsi plus particulièrement l'avantage d'une apparence conservée d'une forme solide classique, notamment celle d'un comprimé, pouvant, si certains patients le désirent, être pris avec un peu d'eau.

Les exemples suivants sont destinés à illustrer la présente invention.

### Exemple 1 : sphéroïdes d'amisulpride obtenus par granulation humide

On prépare des particules d'amisulpride, sous forme de sphéroïdes d'amisulpride, par granulation humide à partir des composants suivants (tableau 1) :

**Tableau 1**

| Composants | Pourcentage en poids |
|---|---|
| Amisulpride ¹ | 90 |
| Povidone K30 ² | 10 |
| Isopropanol | q.s.p. granulation |

| | |
|---|---|
| (R,S)-amisulpride fabriqué par la société Finorga ² Kollidon® commercialisée par BASF | |

On opère de la manière suivante, dans un mélangeur granulateur sècheur (MGS) Zanchetta Roto P50. On mélange d'abord l'amisulpride avec la povidone K30 (liant). Le mélange obtenu est ensuite soumis aux étapes successives suivantes : mouillage avec de l'isopropanol, granulation, sphéronisation et séchage pour éliminer le solvant.

Les sphéroïdes d'amisulpride ainsi obtenus sont calibrés dans une tamiseuse, à une taille comprise entre 125 µm et 500 µm.

On constate un rendement supérieur à 75 % pour ces sphéroïdes d'amisulpride de taille comprise entre 125 µm et 500 µm (le rendement se calcule en effectuant le rapport de la masse des sphéroïdes calibrés à la somme des masses de départ d'amisulpride et des excipients).

### Exemple 2 : sphéroïdes d'amisulpride obtenus par extrusion-sphéronisation

On prépare des sphéroïdes d'amisulpride, sous forme de sphéroïdes d'amisulpride, par extrusion-sphéronisation à partir des composants suivants (tableau 2) :

**Tableau 2**

| Composants | Pourcentage en poids |
|---|---|
| Amisulpride ¹ | 70 |
| Hydroxypropyl cellulose ² | 1 |
| Cellulose microcristalline ³ | 28,5 |
| Laurylsulfate de sodium | 0,5 |
| Eau purifiée | q.s.p. granulation |

| | |
|---|---|
| ¹ (*R*,*S*)-amisulpride fabriqué par Finorga ² Klucel JF® commercialisée par Hercules ³ Avicel PH101 commercialisée par FMC Biopolymer | |

On opère de la manière suivante. Dans un mélangeur granulateur Diosna, on mélange l'amisulpride avec la cellulose microcristalline l'eau purifiée, l'hydroxypropyl cellulose et le laurylsulfate de sodium Le mélange obtenu est ensuite soumis à une granulation selon les étapes suivantes : extrusion dans un extrudeur Fuji Paudal TDG110, sphéronisation dans un sphéroniseur Fuji Paudal Q400 et séchage en étuve pour éliminer l'eau. Les sphéroïdes d'amisulpride ainsi obtenus sont calibrés entre 125 et 500 µm sur tamis.

On constate un rendement supérieur à 95% pour les sphéroïdes d'amisulpride de taille comprise entre 125 µm et 500 µm

### Exemple 3 : sphéroïdes d'amisulpride enrobés par de l'huile de ricin hydrogénée

On prépare des particules d'amisulpride enrobées, sous forme de sphéroïdes enrobés par de l'huile de ricin hydrogénée (CUTINA HR® commercialisée par Sidobre Sinnova) à partir de 1500 g de sphéroïdes d'amisulpride préparés selon l'exemple 1. L'enrobage théorique représente 16,7 % en poids, par rapport au poids des sphéroïdes enrobés.

Les conditions opératoires sont les suivantes, dans un appareil à lit d'air fluidisé Glatt de type GPCG1, de configuration top spray :
- durée totale : 40 min ;
- débit d'air : 105 Nm³/h ;
- température d'air d'atomisation : 124°C;
- pression de pulvérisation : 2 bars.

Le rendement opératoire est supérieur à 95 %. On constate sur des photographies de coupes des sphéroïdes d'amisulpride avant et après enrobage que la couche d'enrobage est homogène et très fine (environ 20 µm d'épaisseur).

Un test gustatif permet de constater que cet enrobage m asque l'amertume d e l'amisulpride.

### Exemple 4 : sphéroïdes d'amisulpride enrobés par du Gelucire®

On prépare des particules d'amisulpride enrobées, sous forme de sphéroïdes enrobés par un mélange de monoester et diesters de polyéthylène glycol avec des mono-, di- et triglycérides (Gelucire 50/02 ® commercialisé par Gattefossé), à partir de 1000 g de sphéroïdes d'amisulpride préparés selon l'exemple 1. L'enrobage théorique représente 16,67 % en poids, par rapport au poids des sphéroïdes enrobés.

Les conditions opératoires sont les suivantes, dans un appareil à lit d'air fluidisé Glatt de type GPCG1, de configuration top spray :
- durée : 40 min ;
- débit d'air : 105 Nm³/h ;
- température d'air d'atomisation : 65°C
- Pression de pulvérisation : 2 bars

Le rendement opératoire est supérieur à 95%.

Un test gustatif permet de constater que cet enrobage masque l'amertume de l'amisulpride.

### Exemple 5 : sphéroïdes d'amisulpride enrobés par de l'éthylcellulose

On prépare des particules d'amisulpride enrobées, sous forme de sphéroïdes d'amisulpride enrobés par une solution à 23,40 % de matière sèche soit 16,54 % d'éthylcellulose à partir de 700 g de sphéroïdes d'amisulpride préparés selon l'exemple 1. L'éthylcellulose est utilisée sous forme d'une dispersion aqueuse contenant
- de l'hydroxypropylcellulose (HPMC),
- de l'Aquacoat® (commercialisée par FMC BioPolymer), et
- du Syloïd® .

On pulvérise cette dispersion sur les sphéroïdes d'amisulpride, dans un appareil à lit d'air fluidisé Glatt de type GPCG1, de configuration Würster.

Le film d'enrobage a la composition suivante (tableau 3) :

**Tableau 3**

| Composants | % en poids de matière sèche |
|---|---|
| Aquacoat® | 78,52 |
| dont éthylcellulose | 70,67 |
| HPMC | 15,73 |
| Syloïd ® | 5,75 |
| **Total :** | **100%** |

Un test gustatif permet de constater que l'amertume de l'amisulpride est masquée avec 22,14 % en poids de cet enrobage (dont 15,64 % d'éthylcellulose), par rapport au poids des sphéroïdes d'amisulpride enrobés.

### Exemple 6 : sphéroïdes d'amisulpride enrobés par un polymère acrylique

On prépare des particules d'amisulpride enrobées, sous forme de sphéroïdes d'amisulpride enrobés par une solution à 23 % de matière sèche soit 15,75 % d'Eudragit E commercialisé par RÖHM §HAAS, à partir de 1000 g de sphéroïdes d'amisulpride préparés selon l'exemple 1.

Le polymère Eudragit E® est utilisé sous forme d'une dispersion aqueuse contenant du stéarate de magnésium, du lauryl sulfate de sodium et du dibutyl sébaçate.
- La dispersion est pulvérisée sur les sphéroïdes d'amisulpride dans un appareil à lit d'air fluidisé Glatt de type GPCG1 de configuration Würster.

Le film d'enrobage a la composition suivante (tableau 4) :

**Tableau 4**

| Composants | % en poids de matière sèche d'enrobage |
|---|---|
| Eudragit E® | 68,5 |
| Lauryl sulfate de sodium | 4,5 |
| Stéarate de magnésium | 17 |
| Dibutyl sébaçate | 10 |
| **Total :** | **100 %** |

Un test gustatif permet de constater que l'amertume de l'amisufpride est masquée avec 14,5 % en poids de cet enrobage (dont 10,25 % de polymère Eudragit E®), par rapport au poids des sphéroïdes d'amisulpride enrobés

### Exemple 7 : comprimé orodispersible d'amisulpride

Dans cet exemple, les particules d'amisulpride enrobées sont les sphéroïdes d'amisulpride enrobés préparés à l'exemple 5.

On prépare des comprimés orodispersibles d'amisulpride par compression directe, après mélange des composants, sur une machine rotative type SVIAC PR12 avec un poinçon 16mm et une force de compression inférieure à 600 daN, ces comprimés ayant ainsi la composition suivante (tableau 5) :

**Tableau 5**

| Composants | Masse (mg) |
|---|---|
| Sphéroïdes d'amisulpride enrobés (exemple 5) | 460 |
| Glycine ¹ | 322 |
| Lactose / PVP / PVP réticulée ² | 276 |
| Crospovidone ³ | 23 |
| Aspartame | 11,5 |
| Acide citrique | 34,5 |
| Stéarate de magnésium | 5,75 |
| Silice colloïdale | 5,75 |
| Arômes | 11,5 |
| **Total :** | **1150 mg** |

| | |
|---|---|
| ¹ Glycine BN 500 ² Ludipress ® ³ _{K}ollidon CL ® | |

## Revendications

1. Composition, pharmaceutique solide pour administration par voie orale du principe actif amisulpride, **caractérisée en ce qu'**elle comprend au moins une particule d'amisulpride enrobée et au moins un excipient pharmaceutiquement acceptable adapté à une administration orodispersible de la composition, cette composition étant sous forme orodispersible.

2. Composition selon la revendication 1, **caractérisée en ce que** ladite au moins une particule d'amisulpride enrobée est constituée d'une particule d'amisulpride présentant à sa surface un enrobage choisi dans le groupe constitué par les enrobages lipidiques et les enrobages polymères.

3. Composition selon la revendication 2, **caractérisée en ce que** ledit enrobage est un enrobage lipidique comprenant une matière lipidique choisie dans le groupe constitué par les mono-, di- et triglycérides d'acides gras, les monoesters et diesters de polyglycérols, les monoesters et diesters de polyéthylène glycol, les esters de propylène glycol, les esters d'acides gras de sucrose, les acides gras et les alcools gras, les cires végétales, les cires animales, les cires de pétrole, les cires synthétiques, et les mélanges de ceux-ci.

4. Composition selon la revendication 3, **caractérisée en ce que** ladite matière lipidique est choisie dans le groupe constitué par l'huile de ricin hydrogénée, ies mélanges de mono-, di- et triglycérides d'acides gras avec des monoesters et diesters de polyéthylène glycol, et les mélanges de ceux-ci.

5. Composition selon la revendication 2, **caractérisée en ce que** ledit enrobage est un enrobage polymère comprenant un polymère choisi dans le groupe constitué par les polymères cellulosiques, les polymères acryliques, les polymères vinyliques, les polymères carboxyvinyliques, et les mélanges de ceux-ci.

6. Composition selon la revendication 5, **caractérisée en ce que** ledit enrobage polymère comprend un polymère cellulosique choisi dans le groupe constitué par l'éthylcellulose, l'hydroxyethylcellulose, l'hydroxypropylcellulose, l'hydroxypropylméthylcellulose, la carboxyméthylcellulose, et les mélanges de ceux-ci.

7. Composition selon la revendication 5, **caractérisée en ce que** ledit enrobage polymère comprend un polymère acrylique choisi dans le groupe constitué par les polymères acryliques, les polymères méthacryliques, les copolymères amonio-méthacrylates, les polyacrylates, les polyméthacrylates, et les mélanges de ceux-ci.

8. Composition selon la revendication 1, **caractérisée en ce que** ladite au moins une particules d'amisulpride enrobée est susceptible d'être obtenue par un procédé comprenant au moins une étape de mlcroencapsulation de particule d'amisulpride.

9. Composition selon l'une quelconque des revendications précédentes, **caractérisée en ce que** ladite au moins une particule d'amisulpride enrobée présente une taille comprise entre 21 µm et 1000 µm.

10. Composition selon l'une quelconque des revendications précédentes, **caractérisée en ce que** ladite au moins une particule d'amisulpride enrobée est constituée d'une particule d'amisulpride présentant à sa surface un enrobage, la proportion d'enrobage étant comprise entre 1 et 50 % en poids, par rapport au poids total de particule d'amisulpride enrobée.

11. Composition selon l'une quelconque des revendications précédentes, **caractérisée en ce que** ladite au moins une particule d'amisulpride enrobée comprend une proportion d'amisulpride comprise entre 40 et 99 % en poids, par rapport au poids total de particule d'amisulpride enrobée.

12. Composition selon l'une quelconque des revendications précédentes, **caractérisée en ce que** ladite au moins une particule d'amisulpride enrobée est constituée d'une particule d'amisulpride présentant à sa surface un enrobage, la particule d'amisulpride présentant une taille comprise entre 20 µm et 700 µm.

13. Composition selon l'une quelconque des revendications précédentes, **caractérisée en ce que** ladite au moins une particule d'amisulpride enrobée est constituée d'une particule d'amisuipride présentant à sa surface un enrobage, ia particule d'amisulpride étant un sphéroïde d'amlsulpride comprenant en outre au moins un excipient de sphéronisation choisi dans le groupe constitué par les agents liants, les agents diluants, les tensioactifs, et les mélanges de ceux-ci.

14. Composition selon la revendication 13, **caractérisée en ce que** ledit au moins un excipient de sphéronisation est choisi dans le groupe constitué par la povidone, l'hydroxypropylcellulose, la cellulose microcristalline, le laurylsulfate de sodium, e t les mélanges de ceux-ci.

15. Composition selon l'une quelconque des revendications 1 à 12, **caractérisée en ce que** ladite au moins une particule d'amisulpride enrobée est constituée d'une particule d'amisulpride présentant à sa surface un enrobage, la particule d'amisulpride étant susceptible d'être obtenue par un procédé comprenant au moins une étape de cristallisation d'amisulpride.

16. Composition selon l'une quelconque des revendications précédentes, **caractérisée en ce que** ledit au moins un excipient pharmaceutiquement acceptable adapté à une administration orodispersible de la composition est choisi dans le groupe constitué par agents désintégrants, les agents de salivation, les agents diluants à propriétés liantes, et les mélanges de ceux-ci.

17. Composition selon la revendication 16, **caractérisée en ce que** ledit au moins un excipient pharmaceutiquement acceptable adapté à une administration orodispersible de la composition est choisi dans le groupe constitue par la crospovidone, la croscarmellose, les amidons et leurs dérivés, les dérivés d'hydroxypropylcellulose faiblement substitués, l'acide alginique et ses sels, l'acide citrique et ses sels, les polyols, et les mélanges de ceux-ci.

18. Composition selon l'une quelconque des revendications précédentes, **caractérisée en ce que** ledit au moins un excipient pharmaceutiquement acceptable adapté à une administration orodispersible de la composition est présent selon une proportion comprise entre 2 et 90 % en poids, par rapport au poids total de la composition.

19. Composition selon l'une quelconque des revendications précédentes, **caractérisée en ce qu'**elle comprend en outre, avec ladite au moins une particule d'amisulpride enrobée et ledit au moins un excipient pharmaceutiquement acceptable adapté à une administration orodispersible de la composition, au moins un excipient pharmaceutiquement acceptable adapté au masquage de goût.

20. Composition selon la revendication 19, **caractérisée en ce que** ledit au moins un excipient pharmaceutiquement acceptable a dapté a u m asquage dégoût est choisi dans le groupe constitué par les arômes naturel, les arômes de synthèse, ies édulcorants de synthèse, et les mélanges de ceux-ci.

21. Composition selon la revendication 19 ou 20, **caractérisée en ce que** ledit au moins un excipient pharmaceutiquemant acceptable adapté au masquage de goût est présent selon une proportion comprise entre 0,1 et 6 % en poids, par rapport au poids total de la composition.

22. Composition selon l'une quelconque des revendications précédentes, **caractérisée en ce qu'**elle comprend en outre au moins un excipient pour forme solide choisi dans le groupe constitué par les agents diluants, les agents lubrifiants, les agents d'écoulement, les agents mouillants, les agents colorants, les agents modificateurs de pH, les agents liants, et les mélanges de ceux-ci.

23. Composition selon la revendication 22, **caractérisée en ce que** ledit au moins un excipient pour forme solide est choisi dans le groupe constitué par la cellulose microcristalline, le lactose, la glycine, le stéarate de magnésium, la silice colloïdale, le polyéthylène glycol, la polyvinylpyrrolidone, les polymères cellulosiques, les polymères acryliques, les huiles végétales, les gommes, la gélatine, et les mélanges de ceux-ci.

24. Composition selon l'une quelconque des revendications précédentes, **caractérisée en ce que** l'amisulpride est choisi dans le groupe constitué par le (*R*,*S*)-amisulpride, les isomères levogyre et dextrogyre de l'amisulpride, les dérivés de l'amlsulpride ou de l'un de ses isomères levogyre et dextrogyre, ces dérivés étant choisis parmi les sels d'addition avec un acide pharmaceutiquement acceptable, les sels d'ammonium quaternaire et les oxydes, de l'amisulpride ou de l'un de ses isomères levogyre et dextrogyre, et les mélanges de ceux-ci.

25. Composition selon l'une quelconque des revendications précédentes, **caractérisée en ce que** l'amisulpride est le (*R*,*S*)-amisulpride.

26. Composition selon l'une quelconque des revendications précédentes, **caractérisée en ce que** l'amisulpride est présent selon une proportion comprise entre 5 et 80% en poids, par rapport au poids total de la composition.

27. Composition selon l'une quelconque des revendications précédentes, **caractérisée en ce qu'**elle comprend, par rapport au poids total de la composition :
- 1 0 à 40 % en poids d e (R,S)-amisulpride sous forme d'au moins une particule d'amisulpride enrobée;
- 3 à 85% en poids d'excipient pour administration orodispersible;
- 1 à 5 % en poids d'excipient pour masquage de goût ;
- 1 à 86 % en poids d'excipient pour forme solide.

28. Composition selon l'une quelconque des revendications précédentes, **caractérisée en ce qu'**elle se présente sous forme d'un comprimé orodispersible.

29. Composition selon l'une quelconque des revendications précédentes, **caractérisée en ce qu'**elle se présente sous forme d'un comprimé orodispersible comprenant de l'amisulpride selon un dosage compris entre 50 et 800 mg.

30. Composition selon l'une quelconque des revendications précédentes, **caractérisée en ce qu'**elle est à libération immédiate d'amisulpride.

## Patentansprüche

1. Feste pharmazeutische Zusammensetzung zur oralen Verabreichung des Wirkstoffs Amisulprid, **dadurch gekennzeichnet, dass** sie mindestens ein beschichtetes Amisulprid-Teilchen und mindestens einen pharmazeutisch unbedenklichen Hilfsstoff, der für eine orodispersible Verabreichung der Zusammensetzung geeignet ist, umfasst, wobei diese Zusammensetzung in orodispersibler Form vorliegt.

2. Zusammensetzung nach Anspruch 1, **dadurch gekennzeichnet, dass** das mindestens eine beschichtete Amisulprid-Teilchen aus einem Amisulprid-Teilchen, das an seiner Oberfläche eine Beschichtung aus der Gruppe bestehend aus Lipidbeschichtungen und Polymerbeschichtungen aufweist, besteht.

3. Zusammensetzung nach Anspruch 2, **dadurch gekennzeichnet, dass** es sich bei der Beschichtung um eine Lzpidbeschichtung, die ein Lipidmaterial aus der Gruppe bestehend aus Fettsäuremonoglyceriden, -diglyceriden und -triglyceriden, Polyglycerinmonoestern und -diestern, Polyethylenglykolmonoestern und -diestern, Propylenglykolestern, Saccharosefettsäureestern, Fettsäuren und Fettalkoholen, pflanzlichen Wachsen, tierische Wachsen, Erdölwachsen und synthetischen Wachsen und Mischungen davon umfasst, handelt.

4. Zusammensetzung nach Anspruch 3, **dadurch gekennzeichnet, dass** das Lipidmaterial aus der Gruppe bestehend aus hydriertem Ricinusöl und Mischungen von Fettsäuremonoglyceriden, -diglyceriden und -triglyceriden mit Polyethylenglykolmonoestern und -diestern und Mischungen davon ausgewählt ist.

5. Zusammensetzung nach Anspruch 2, **dadurch gekennzeichnet, dass** es sich bei der Beschichtung um eine Polymerbeschichtung, die ein Polymer aus der Gruppe bestehend aus Cellulosepolymeren, Acrylpolymeren, Vinylpolymeren, Carboxyvinylpolymeren und Mischungen davon umfasst, handelt.

6. Zusammensetzung nach Anspruch 5, **dadurch gekennzeichnet, dass** die Polymerbeschichtung ein Cellulosepolymer aus der Gruppe bestehend aus Ethylcellulose, Hydroxyethylcellulose, Hydroxypropylcellulose, Hydroxypropylmethylcellulose, Carboxymethylcellulose und Mischungen davon umfasst.

7. Zusammensetzung nach Anspruch 5, **dadurch gekennzeichnet, dass** die Polymerbeschichtung ein Acrylpolymer aus der Gruppe bestehend aus Acryl-polymeren, Methacrylpolymeren, Ammoniomethacrylat-Copolymeren, Polyacrylaten, Polymethacrylaten und Mischungen davon umfasst.

8. Zusammensetzung nach Anspruch 1, **dadurch gekennzeichnet, dass** das mindestens eine beschichtete Amisulprid-Teilchen nach einem Verfahren erhältlich ist, das mindestens einen Schritt der Mikroverkapselung des Amisulprid-Teilchens umfasst.

9. Zusammensetzung nach einem der vorhergehenden Ansprüche, dadurch gekenntzeichnet, dass das mindestens eine beschichtete Amisulprid-Teilchen eine Größe zwischen 21 µm und 1000 µm aufweist.

10. Zusammensetzung nach einem der vorhergehenden Ansprüche, **dadurch gekennzeichnet, dass** das mindestens eine beschichtete Amisulprid-Teilchen aus einem Amisulprid-Teilchen, das an seiner Oberfläche eine Beschichtung aufweist, besteht, wobei der Beschlchtungsanteil zwischen 1 und 50 Gew.-%, bezogen auf das Gesamtgewicht des beschichteten Amisulprid-Teilchens, liegt.

11. Zusammensetzung nach einem der vorhergehenden Ansprüche, **dadurch gekennzeichnet, dass** das mindestens eine beschichtete Amisulprid-Teilchen einen Amisulprid-Anteil zwischen 40 und 99 Gew.-%, bezogen auf das Gesamtgewicht des beschichteten Amisulprid-Teilchens, aufweist.

12. Zusammensetzung nach einem der vorhergehenden Ansprüche, **dadurch gekennzeichnet, dass** das mindestens eine beschichtete Amisulprid-Teilchen aus einem Amisulprid-Teilchen, das an seiner Oberfläche eine Beschichtung aufweist, besteht, wobei das Amisulprid-Teilchen eine Größe zwischen 20 µm und 700 µm aufweist.

13. Zusammensetzung nach einem der vorhergehenden Ansprüche, **dadurch gekennzeichnet, dass** das mindestens eine beschichtete Amisulprid-Teilchen aus einem Amisulprid-Teilchen, das an seiner Oberfläche eine Beschichtung aufweist, besteht, wobei es sich bei dem Amisulprid-Teilchen um ein Amisulprid-Sphäroid handelt, das außerdem mindestens einen Sphäronisierungshilfsstoff aus der Gruppe bestehend aus Bindemitteln, Verdünnungsmitteln, Tensiden und Mischungen davon umfasst.

14. Zusammensetzung nach Anspruch 13, **dadurch gekennzeichnet, dass** der mindestens eine Sphäronisierungshilfsstoff aus der Gruppe bestehend aus Povidon, Hydroxypropylcellulose, mikrokristalliner Cellulose, Natriumlaurylsulfat und Mischungen davon ausgewählt ist.

15. Zusammensetzung nach einem der Ansprüche 1 bis 12, **dadurch gekennzeichnet, dass** das mindestens eine beschichtete Amisulprid-Teilchen aus einem Amisulprid-Teilchen, das an seiner Oberfläche eine Beschichtung aufweist, besteht, wobei das Amisulprid-Teilchen nach einem Verfahren erhältlich ist, das mindestens einen Schritt der Kristallisation von Amisulprid umfasst.

16. Zusammensetzung nach einem der vorhergehenden Ansprüche, **dadurch gekennzeichnet, dass** der mindestens eine pharmazeutisch unbedenkliche Hilfsstoff, der für eine orodispersible Verabreichung der Zusammensetzung geeignet ist, aus der Gruppe bestehend aus Sprengmitteln, Speichelbildungsmitteln, Verdünnungsmitteln mit Bindungseigenschaften und Mischungen davon ausgewählt ist.

17. Zusammensetzung nach Anspruch 16, **dadurch gekennzeichnet, dass** der mindestens eine pharmazeutisch unbedenkliche Hilfsstoff, der für eine orodispersible Verabreichung der Zusammensetzung geeignet ist, aus der Gruppe bestehend aus Crospovidon, Croscarmellose, Stärken und Stärkederivaten, gering substituierten Hydroxypropylcellulosederivaten, Alginsäure und Salzen davon, Citronensäure und Salzen davon, Polyolen und Mischungen davon ausgewählt ist.

18. Zusammensetzung nach einem der vorhergehenden Ansprüche, **dadurch gekennzeichnet, dass** der mindestens eine pharmazeutisch unbedenkliche Hilfsstaff, der für eine orodispersible Verabreichung der Zusammensetzung geeignet ist, in einem Anteil zwischen 2 und 90 Gew.-%, bezogen auf das Gesamtgewicht der Zusammensetzung, vorliegt.

19. Zusammensetzung nach einem der vorhergehenden Ansprüche, **dadurch gekennzeichnet, dass** sie außerdem neben dem mindestens einen beschichteten Amisulprid-Teilchen und dem mindestens einen pharmazeutisch unbedenklichen Hilfsstoff, der für eine orodispersible Verabreichung der Zusammensetzung geeignet ist, mindestens einen pharmazeutisch unbedenklichen Hilfsstoff, der zur Geschmacksmaskierung geeignet ist, umfasst.

20. Zusammensetzung nach Anspruch 19, **dadurch gekennzeichnet, dass** der mindestens eine pharmazeutisch unbedenkliche Hilfsstoff, der zur Geschmacksmaskierung geeignet ist, aus der Gruppe bestehend aus natürliche Geschmacksstoffen, synthetischen Geschmacksstoffen, synthetischen Süßungsmitteln und Mischungen davon ausgewählt ist.

21. Zusammensetzung nach Anspruch 19 oder 20, **dadurch gekennzeichnet, dass** der mindestens eine pharmazeutisch unbedenkliche Hilfsstoff, der zur Geschmacksmaskierung geeignet ist, in einem Anteil zwischen 0,1 und 6 Gew.-%, bezogen auf das Gesamtgewicht der Zusammensetzung, vorliegt.

22. Zusammensetzung nach einem der vorhergehenden Ansprüche, **dadurch gekennzeichnet, dass** sie außerdem mindestens einen Hilfsstoff für eine feste Form, der aus der Gruppe bestehend aus Verdünnungsmitteln, Gleitmitteln, Fließmitteln, Netzmitteln, Farbmitteln, Mitteln zur Modifizierung des pH-Werts, Bindemitteln und Mischungen davon ausgewählt ist, umfasst.

23. Zusammensetzung nach Anspruch 22, **dadurch gekennzeichnet, dass** der mindestens eine Hilfsstoff für eine feste Form aus der Gruppe bestehend aus mikrokristalliner Cellulose, Lactose, Glycin, Magnesiumstearat, kolloidalem Siliciumdioxid, Polyethylenglykol, Polyvinylpyrrolidon, Cellulosepolymeren, Acrylpolymeren, pflanzlichen Ölen, Gummen, Gelatine und Mischungen davon ausgewählt ist.

24. Zusammensetzung nach einem der vorhergehenden Ansprüche, **dadurch gekennzeichnet, dass** das Amisulprid aus der Gruppe bestehend aus (*R*,*S*)-Amisulprid, den linksdrehenden und rechtsdrehenden Isomeren von Amisulprid, Derivaten von Amisulprid oder einem seiner linksdrehenden oder rechtsdrehenden Isomere, wobei diese Derivate aus Additionssalzen mit einer pharmazeutisch unbedenklichen Säure, quaternären Ammoniumssalzen und Oxiden von Amisulprid oder einem seiner linksdrehenden oder rechtsdrehenden Isomere ausgewählt sind, und Mischungen davon ausgewählt ist.

25. Zusammensetzung nach einem der vorhergehenden Ansprüche, **dadurch gekennzeichnet, dass** es sich bei dem Amisulprid um (*R*,*S*)-Amisulprid handelt.

26. Zusammensetzung nach einem der vorhergehenden Ansprüche, **dadurch gekennzeichnet, dass** das Amisulprid in einem Anteil zwischen 5 und 80 Gew.-%, bezogen auf das Gesamtgewicht der Zusammensetzung, vorliegt.

27. Zusammensetzung nach einem der vorhergehenden Ansprüche, **dadurch gekennzeichnet, dass** sie, bezogen auf das Gesamtgewicht der Zusammensetzung:
- 10 bis 40 Gew.-% (*R*,*S*)-Amisulprid in Form mindestens eines beschichteten Amisulprid-Teilchens;
- 3 bis 85 Gew.-% Hilfsstoff für orodispersible Verabreichung;
- 1 bis 5 Gew.-% Hilfsstoff zur Geschmackes markierung;
- 1 bis 86 Gew.-% Hilfsstoff für eine feste Form umfasst.

28. Zusammensetzung nach einem der vorhergehenden Ansprüche, **dadurch gekennzeichnet, dass** sie in Form einer orodispersiblen Tablette vorliegt.

29. Zusammensetzung nach einem der vorhergehenden Ansprüche, **dadurch gekennzeichnet, dass** sie in Form einer orodispersiblen Tablette, die Amisulprid in einer Dosierung zwischen 50 und 800 mg umfasst, vorliegt.

30. Zusammensetzung nach einem der vorhergehenden Ansprüche, **dadurch gekennzeichnet, dass** sie zur sofortigen Freisetzung von Amisulprid vorgesehen ist.

## Claims

1. Solid pharmaceutical composition for oral administration of the active principle amisulpride, **characterized in that** it comprises at least one coated amisulpride particle and at least one pharmaceutically acceptable excipient suitable for an orodispersible administration of the composition, this composition being in orodispersible form.

2. Composition according to Claim 1, **characterized in that** said at least one coated amisulpride particle consists of an amisulpride particle having at its surface a coating chosen from the group consisting of lipid coatings and polymer coatings.

3. Composition according to Claim 2, **characterized in that** said coating is a lipid coating comprising a lipid material chosen from the group consisting of fatty acid mono-, di- and triglycerides, polyglyceryl monoesters and diesters, polyethylene glycol monoesters and diesters, propylene glycol esters, fatty acid esters of sucrose, fatty acids and fatty alcohols, plant waxes, animal waxes, petroleum waxes and synthetic waxes, and mixtures thereof.

4. Composition according to Claim 3, **characterized in that** said lipid material is chosen from the group consisting of hydrogenated castor oil and mixtures of fatty acid mono-, di- and triglycerides with polyethylene glycol monoesters and diesters, and mixtures thereof.

5. Composition, according to Claim 2, **characterized in that** said coating is a polymer coating comprising a polymer chosen from the group consisting of cellulose-based polymers, acrylic polymers, vinyl polymers and carboxyvinyl polymers, and mixtures thereof.

6. Composition according to Claim 5, **characterized in that** said polymer coating comprises a cellulose-based polymer chosen from the group consisting of ethylcellulose, hydroxyethylcellulose, hydroxypropylcellulose, hydroxypropylmethylcellulose and carboxymethylcellulose, and mixtures thereof.

7. Composition according to Claim 5, **characterized in that** said polymer coating comprises an acrylic polymer chosen from the group consisting of acrylic polymers, methacrylic polymers, ammoniomethacry7.ate copolymers, polyacrylates and polymethacrylates, and mixtures thereof.

8. Composition according to Claim 1, **characterized in that** said at least one coated amisulpride particle may be obtained via a process comprising at least one step of microencapsulation of the amisulpride particle.

9. Composition according to any one of the preceding claims, **characterized in that** said at least one coated amisulpride particle is between 21 µm and 1000 µm in size.

10. Composition according to any one of the preceding claims, **characterized in that** said at least one coated amisulpride, particle consists of an amisulpride particle having a coating at its surface, the proportion of coating being between 1% and 50% by weight relative to the total weight of coated amisulpride particle.

11. Composition according to any one of the preceding claims, **characterized in that** said at least one coated amisulpride particle comprises a proportion of amisulpride of between 40% and 99% by weight relative to the total weight of coated amisulpride particle.

12. Composition according to any one of the preceding claims, **characterized in that** said at least one coated amisulpride particle consists of an amisulpride particle having a coating at its surface, the amisulpride particle being between 20 µm and 700 µm in size.

13. Composition according to any one of the preceding claims, **characterized in that** said at least one coated amisulpride particle consists of an amisulpride particule having a coating at its surface, the amisulpride particle being an amisulpride spheroid also comprising at least one spheronization excipient chosen from the group consisting of binders, diluents and surfactants, and mixtures thereof.

14. Composition according to Claim 13, **characterized in that** said at least one spheronization excipient is chosen from the group consisting of povidone, hydroxypropylcellulose, microcrystalline cellulose and sodium lauryl sulfate, and mixtures thereof.

15. Composition according to any one of Claims 1 to 12, **characterized in that** said at least one coated amisulpride particle consists of an amisulpride particle having a coating at its surface, the amisulpride particle possibly being obtained via a process comprising at least one step of crystallization of amisulpride.

16. Composition according to any one of the preceding claims, **characterized in that** said at least one pharmaceutically acceptable excipient suitable for orodispersible administration of the composition is chosen from the group consisting of disintegrants, salivation agents and diluents with binding properties, and mixtures thereof.

17. Composition according to Claim 16, **characterized in that** said at least one pharmaceutically acceptable excipient suitable for orodispersible administration of the composition is chosen from the group consisting of crospovidone, croscarmellose, starches and derivatives thereof, sparingly substituted hydroxypropylcellulose derivatives, alginic acid and its salts, citric acid and its salts, and polyols, and mixtures thereof.

18. Composition according to any one of the preceding claims, **characterized in that** said at least one pharmaceutically acceptable excipient suitable for orodispersible administration of the composition is present in a proportion of between 2% and 90% by weight relative to the total weight of the composition.

19. Composition according to any one of the preceding claims, **characterized in that** it also comprises, with said at least one coated amisulpride particle and said at least one pharmaceutically acceptable excipient suitable for orodispersible administration of the composition, at least one pharmaceutically acceptable excipient suitable for taste masking.

20. Composition according to Claim 19, **characterized in that** said at least one pharmaceutically acceptable excipient suitable for taste masking is chosen from the group consisting of natural flavorings, synthetic flavorings and synthetic sweeteners, and mixtures thereof.

21. Composition according to Claim 19 or 20, **characterized in that** said at least one pharmaceutically acceptable excipient suitable for taste masking is present in a proportion of between 0.1% and 6% by weight relative to the total weight of the composition.

22. Composition according to any one of the preceding claims, **characterized in that** it also comprises at least one excipient for a solid form, chosen from the group consisting of diluents, lubricants, flow agents, wetting agents, dyes, pH regulators and binders, and mixtures thereof.

23. Composition according to Claim 22, **characterized in that** said at least one excipient for a solid form is chosen from the group consisting of microcrystalline cellulose, lactose, glycine, magnesium stearate, colloidal silica, polyethylene glycol, polyvinylpyrrolidone, cellulose-based polymers, acrylic polymers, plant oils, gums and gelatin, and mixtures thereof.

24. Composition according to any one of the preceding claims, **characterized in that** the amisulpride is chosen from the group consisting of (R,S)-amisulpride, the levorotatory and dextrorotatory isomers of amisulpride, amisulpride derivatives or derivatives of one of its levorotatory and dextrorotatory isomers, these derivatives being chosen from the addition salts with a pharmaceutically acceptable acid, the quaternary ammonium salts and the oxides, of amisulpride or of one of its levorotatory and dextrorotatory isomers, and mixtures thereof.

25. Composition according to any one of the preceding claims, **characterized in that** the amisulpride is (*R*,*S*)-amisulpride.

26. Composition according to any one of the preceding claims, **characterized in that** the amisulpride is present in a proportion of between 5% and 80% by weight relative to the total weight of the composition.

27. Composition according to any one of the preceding claims, **characterized in that** it comprises, relative to the total weight of the composition:
- 10% to 40% by weight of (*R*,*S*)-amisulpride in the form of at least one coated amisulpride, particle;
- 3% to 85% by weight of excipient for orodispersible administration;
- 1% to 5% by weight of excipient for taste masking;
- 1% to 86% by weight of excipient for a solid form.

28. Composition according to any one of the preceding claims, **characterized in that** it is in the form of an orodispersible tablet.

29. Composition according to any one of the preceding claims, **characterized in that** it is in the form of an orodispersible tablet comprising amisulpride in a dosage of between 50 and 800 mg.

30. Composition according to any one of the preceding claims, **characterized in that** it gives immediate release of amisulpride.
